# EUROPEAN PATENT SPECIFICATION

(11) **EP 1 032 835 B1**
(45) Date of publication and mention of the grant of the patent: **12.01.2005**
(21) Application number: 98955751.7
(22) Date of filing: 23.11.1998
(51) Int. Cl.: G01N 33/543

(54) **IMPROVEMENTS IN OR RELATING TO DISPLACEMENT ASSAYS**
VERBESSERUNGEN AN ODER IN BEZUG AUF VERSCHIEBUNGSTESTS
AMELIORATIONS APPORTEES AUX ANALYSES DE DEPLACEMENT

(30) Priority: 21.11.1997 EP 97309409
(43) Date of publication of application: 06.09.2000
(73) Proprietor: Inverness Medical Switzerland GmbH, 6300 Zug (CH)
(72) Inventor: BADLEY, Robert, Andrew, Bedford MK44 1PL (GB); BERRY, Mark, John, Northamptonshire NN10 8LY (GB); HOWELL, Stephen, Northamptonshire NN10 8LX (GB)
(74) Representative: Lipscombe, Martin John
(86) International application number: PCT/GB1998/003483
(87) International publication number: WO 1999/027368

(56) References cited:
- WO-A-90/06503
- WO-A-91/05262
- WO-A-92/18867
- WO-A-93/21530
- WO-A-97/27474
- WO-A-97/44664
- US-A- 5 514 558

## Description

### Field of the Invention

This invention relates to a method of performing an assay to detect the presence of a substance of interest in a sample.

### Background of the Invention

Various assays for detecting the presence and/or amount of a substance of interest in a sample are known. One particular format of assay is the "displacement" assay in which, conventionally. the presence of an analyte of interest in a sample causes the displacement of a labelled substance (either a labelled binding partner or a labelled ligand) from a pre-existing binding partner/ligand complex. Generally speaking the amount of displaced labelled substance will be proportional to the concentration of the analyte of interest. Alternatively, one may employ "competition" assays, in which there is competition between the analyte of interest and a labelled competitor (such as labelled analyte or analogue) for binding to a limiting number of binding sites.

One particular assay is disclosed in WO 91/05262 (University of Michigan), which relates to an assay device, typically comprising a disposable test strip, wherein the presence of an analyte of interest in a test solution with which the test device is contacted, is indicated by a signal (such as a colour) at a distinct location on the device. and the absence of analyte is indicated by the same signal appearing at a different location.

As disclosed in WO91/05262 a first binding means (such as an immobilised antibody) is provided at one location on the test stick, and has binding specificity for the analyte of interest. A "heterobifunctional complex", comprising the analyte covalently coupled to a "signal generating molecule", is reversibly bound to the first binding means. In the presence of free analyte of interest in the sample, heterobifunctional complex is released from the first binding means and transported via "fluid conducting means" (typically a capillary test stick) to a second binding means. having binding specificity for the signal generating molecule, such that the complex is captured. Typically the signal generating molecule is an enzyme (eg. horseradish peroxidase) such that, when the test stick is dipped into appropriate substrate solution, coloured product (the "signal") forms at the first binding means if no analyte was present in the test sample, or at the second binding means if analyte was present. The signal generating molecule may also be a fluorescent molecule or ultraviolet-absorbing agent.

In this sort of device, there is no interaction between the signal generating molecule and the binding means, beyond simple capture of the complex comprising the signal generating molecule. Thus, the signal generating molecule (the enzyme) is inherently capable of generating a signal (coloured product) whether or not it is released from the first binding means, and the act of capture of the signal generating molecule plays no part in the process of signal generation.

One relatively recent development in relation to assays is the use of devices which employ surface plasmon resonance (SPR). This phenomenon has now been described in several publications and is the basis of evanescent wave biosensors (for a review, see Hutchinson 1995, Molecular Biotechnology 3, 47-54, and references therein).

In summary, light incident on an interface between two media of different refractive indices will, at a specific angle of incidence, generate a resonant "evanescent" wave. The resonance is extremely sensitive to changes in the refractive index of the media. A change in the refractive index causes resonance to occur at a new angle of incidence. The change in refractive index is caused by mass binding to a thin (typically gold) film at the interface between the two media: the change in refractive index is proportional to the mass bound to the gold film.

Examples of such devices which utilise the evanescent wave phenomenon include the BIA lite™ and BIA core™ machines sold by Biacore AB (Sweden), the IA Sys™ device sold by Fisons (UK), and the BIOS-1 device sold by Artificial Sensor Instruments (Zurich, Switzerland).

WO 92/18867 discloses an assay for an analyte of interest, the assay involving the displacement of a second reagent from a first reagent immobilised at a displacement surface, said displacement occurring in response to the presence of the analyte of interest, the displaced second reagent then causing a reaction at a detection surface which alters a third reagent immobilised in the detection surface, and wherein the alteration of the third reagent is detected by means of SPR.

WO 92/18867 is primarily concerned with the assay of analytes (such as haptens) of low molecular weight (page 1, lines 4-6) and addresses a lack of sensitivity in assaying such analytes which is apparent in the prior art (page 2, lines 1-17).

Typically, in WO 92/18867 the first reagent is an analogue of the analyte of interest, and the second reagent is an antibody specific for the analyte. Alternatively, the first reagent is an antibody specific for the analyte and the second reagent is a conjugate comprising an analogue (of the low molecular weight analyte) and a high molecular weight protein. The prior art teaches (page 3, lines 6-10) that:
"An analogue of the analyte is a substance which competes with the analyte for binding to a specific binder thereof. Often the analogue structure will be arranged to be as near as possible or even completely identical to the analyte".

Accordingly WO 92/18867 teaches that the analogue and the analyte should be structurally the same as "near as possible", and there is no disclosure or suggestion that there should be a difference in the relative binding affinities between the first reagent, the second reagent, and the analyte of interest.

### Summary of the Invention

In a first aspect the invention provides a method of detecting the presence of an analyte of interest in the sample, the method comprising the steps of: providing a first surface having reversibly immobilised thereon a displaceable moiety; exposing the first surface to a sample comprising the analyte of interest, the analyte of interest specifically displacing the displaceable moiety from the first surface in an affinity-related manner; causing the displaceable moiety displaced from the first surface to contact a second surface bearing a capture moiety which specifically binds to the displaceable moiety, so as to capture the displaceable moiety on the second surface, said capture generating a detectable signal; and detecting the signal, wherein said detection is performed by means other than surface plasmon resonance and wherein the displaceable moiety cannot generate the signal which is detected in the assay unless and until the displaceable moiety is captured on the second surface.

The method of the invention may be used to detect the presence of any analyte of interest. Examples of such analytes include antibodies (which may be useful in diagnostic methods), nucleic acids (DNA and/or RNA), hormones (of both the protein and the steroid variety), growth factors and the like. Of particular interest are sex and/or fertility hormones and analogues thereof, such as estrone-3-glucuronide (E3G), progestogen-3-glucuronide (P3G), human chorionic gonadotrophin (hCG), luteinising hormone (LH) and follicle stimulating hormone (FSH), wherein detection of such analytes may be useful in pregnancy testing. Conveniently the analyte is present in a liquid sample, especially a mammalian body fluid, such as blood, serum, urine or saliva.

The method of the invention may be used qualitatively (e.g. to detect the presence of the analyte of interest) or, more preferably, quantitatively such that the amount of analyte present may be determined.

The displaceable moiety and the capture moiety advantageously comprise members of a specific binding pair, such that when the displaceable moiety is displaced from the first surface it may readily be captured specifically at the second surface by the capture moiety. Many specific binding pairs are known to those skilled in the art and include, for example, antigens/antibodies, complementary (i.e. specific base pairing) nucleic acid strands, enzymes/substrates, lectins/sugars, hormones/receptors, and the like.

Generally the first surface will comprise a plurality of the displaceable moieties. Typically the displaceable moieties form a coating or layer attached to the first surface. The displaceable moiety may be directly attached to the first surface. However, more generally, the displaceable moiety will be indirectly attached to the first surface via an intervening molecule, which arrangement facilitates accomplishment of the desired degree of reversible immobilisation of the displaceable moiety.

Typically the intervening molecule will be immobilised to the first surface, by any of a number of conventional chemical techniques, whilst the displaceable moiety is relatively weakly attached to the intervening molecule (and thus reversibly immobilised upon the first surface). Desirably the intervening molecule will be an analogue (such as a mimotope, discussed below) of the analyte of interest, the arrangement being such that the binding affinity of the displaceable moiety (or, in other embodiments, of the intervening molecule) for the intervening molecule will be relatively low (eg. 1-50%, more typically 1-10%, preferably 1-5%) compared to the binding affinity of the displaceable moiety for the analyte of interest. Accordingly, in the presence of the analyte of interest, the analyte will tend specifically to displace the displaceable moiety from the first surface. The method of the invention comprises specific, affinity-related displacement of the displaceable moiety, such that the analyte of interest causes quantitative displacement of the displaceable moiety. The relative affinities are desirably selected such that the presence of a small amount of analyte is sufficient to displace a reasonable amount of the displaceable moiety, so that the assay method of the inevntion is of high sensitivity.

In preferred embodiments the displaceable moiety comprises an immunoglobulin molecule or antigen-binding derivative thereof (such as a Fv, Fab, Fab₂, scFv, heavy chain variable regions ["Hcv]", such as those obtainable from Ilamas and camels] and the like). Particularly convenient examples are monoclonal antibodies, bispecific antibodies and "Diabodies", and fusion (chimeric) polypeptides comprising an immunoglobulin molecule or antigen-binding derivative thereof. Fusion proteins may comprise, for example, an immunoglobulin molecule (or antigen-binding derivative thereof) joined to one or more additional functional domains, such as an enzyme (e.g. catalase or glucose oxidase), or a portion which facilitates specific capture by the capture moiety (e.g. a mimotope - i.e. a structural analogue of an epitope reecognised by an antibody, or some other molecule such as biotin, avidin or other member of a specific binding pair, wherein the capture moiety comprises the reciprocal member of the specific binding pair), or facilitates reversible immobilisation on the first surface.

In general, the capture moiety will comprise the reciprocal member of a specific binding pair (i. e. the reciprocal member is that which binds to the member of the pair normally present in the displaceable moiety), and conveniently will be immobilised on the second surface. Conventional chemical methods, well known to those skilled in the art, may be used to effect such immobilisation. It will be understood that the term "immobilisation" in relation to the capture moiety, is intended to indicate that the capture moiety remains bound to the second surface under normal assay conditions, although when an assay has been completed harsh chemical treatment (e.g. very high or very low pH, or the use of chaotropic agents) might be possible to remove the capture moiety from the second surface, such that the surface may be coated with a different capture moiety, enabling performance of the method of the invention for a different analyte of interest. In general however, such treatment is not necessary as one can normally modify the first surface appropriately for use in connection with another analyte.

An important feature of the present invention is the nature of the second surface. In the present invention, the second surface takes what may be described as an "active" part in the signal generation, which contrasts with conventional prior art assays, in which the surface simply acts as in a passive manner as a support. In the present invention, it is the act of capture of the displacement moiety at the second surface which leads indirectly or (preferably) directly to the generation of the detectable signal. The displaceable moiety cannot generate the detectable signal which is actually observed or monitored in the assay unless and until it is captured at the second surface, and generally signal generation depends upon an interaction of the displaceable moiety with the second surface.

The present invention provides a number of advantages over prior art assay methods and devices. Firstly, the invention allows for the use of a generic assay method and device which can readily be modified for use in the detection of any analyte of interest: the same second surface can be used (with the same type of signal generated and detected), only the relatively cheap, simple first surface need be altered, so that the displaceable moiety is displaced in response to the presence of the appropriate analyte. Secondly, the assay method, incorporating a specific displacement step, allows for the testing of samples which are heterogeneous (e.g. include particulate matter such as food particles, blood cells or bacteria) or crude and contain many contaminants (e.g. serum, urine etc.). In addition, the assay of the present invention includes both a specific displacement step, and a specific capture step. This means that the overall specificity of the assay is effectively the mathematical product of the specificities of the displacement and capture steps, giving a far higher specificity than that conferred by most prior art assays. Moreover, this is achievable in a format which, as far as the user is concerned, is essentially a one-step process: the person performing the assay simply has to contact the sample with the first surface, which sets in chain the series of events which lead to the generation of the detectable signal.

In certain embodiments the second surface forms the detection surface of an evanescent or acoustic wave-type biosensor (e. g. as disclosed in EP 0 416 730; EP 0 517 001; or WO 97/04314) such that capture of the displaceable moiety at the surface causes an increase in mass which generates a detectable change in the signal at the second surface, (i.e. capture of the displaceable moiety regulates or modifies a pre-existing signal in a detectable manner).

In some embodiments, capture of the displaceable moiety at the second surface inhibits, in a detectable manner, the generation of a signal, such inhibition constituting the "detectable signal". For example, capture of the displaceable moiety may inhibit a reaction on-going at the second surface: in one embodiment, the second surface comprises a plurality of enzyme molecules which catalyse a reaction producing a detectable (e.g. coloured) product, and the displaceable moiety comprises an immunoglobulin molecule with specific binding activity for the enzyme, such that binding of the displaceable moiety to the enzyme inhibits its catalytic activity (or modifies it in some other way e.g. by binding to an allosteric site), thus producing a detectable change in behaviour of the enzyme.

In alternative preferred embodiments, an evanescent or acoustic wave propagated at the second surface interacts with the displaceable moiety to generate an entirely new signal, rather than the modulation of a pre-existing signal. For example, the displaceable moiety may comprise a fluorophor which is stimulated by the evanescent wave at the second surface to generate fluorescence: because of the short "range" of the evanescent wave, the fluorophor cannot be stimulated unless the displaceable moiety is captured at the second surface. Further, a degree of specificity is involved in the interaction as the fluorophore and wavelength of the evanescent wave are selected such that the evanescent wave has appropriate characteristics to cause excitation of the fluorophor.

In a preferred embodiment, the capture of the displaceable moiety by the capture moiety modulates an electrochemical property of the capture moiety, which modulation comprises the detectable signal. In particular, the capture moiety typically comprises an immunoglobulin molecule (preferably an antibody) or an antigen-binding derivative thereof, immobilised to an electrically conductive support, wherein binding of the displaceable moiety to the capture moiety alters an electrochemical property of the capture moiety, generating a signal which may be detected by, for example, amperometric, voltametric or coulometric means. Preferably the capture moiety and displaceable moiety are selected such that capture of the displaceable moiety directly alters an electrochemical property (e.g. a redox potential) of the capture moiety.

In some embodiments of the invention, the distance between the first surface and the second surface is very small (e.g. in the range 50 µm to 5mm), so that simple diffusion of the displaceable moiety (such as a displaced antibody molecule) between the first and second surfaces is sufficient to provide reasonably rapid response times, where these are required. The distance between the first and second surface may be even smaller than that suggested above in devices which utilise "nanotechnology" principles: in such devices the gap may be as small as the size of a single molecule (e.g. the 5 to 50nm of a typical immunoglobulin molecule or bispecific antibody), in order to minimise the times of mass transport between the first and second surface.

In alternative embodiments, where diffusion of the displaceable moiety from the first to the second surface is not sufficient, it will be necessary to provide some sort of "motive force" to convey the displaceable moiety from the first surface to the second surface. This could be by capillary flow, or by bulk fluid flow (using a pump means) or, for example, by electrostatic attraction between the displaceable moiety and the second surface.

The first and second surfaces may be provided on separate respective first and second solid supports, which facilitates the inclusion of filter means as described below. Alternatively, the first and second surfaces may be provided on a single solid support. The solid support may be substantially planar, or particulate, and may be porous or impermeable. Where both the first and second surfaces are provided on a single support, the surfaces may be present as distant portions of the support, or may be present as closely adjacent portions of the support (e.g. with one surface surrounded, in two dimensions, by the other surface, or with the two surfaces intimately interdigitated). Examples of particulate supports include beads comprising polymeric substances (e.g. polysaccharides such as dextran; or latex or other material) which are well known to those skilled in the art. Examples of substantially planar supports include the sensor chips used in connection with known evanescent or acoustic wave type biosensors.

It may be preferred in some embodiments to position a filter means between the first and second surface such that particulate materials, present in some samples, may be removed. This may be desirable as such material may have detrimental effects on the assay result (e.g. due to non-specific binding of particulate material at the second surface). Suitable micropore filter membranes are well-known to those skilled in the art.

Generally apparatus for performing the method will comprise: a first surface, having reversibly immobilised thereon a displaceable moiety, wherein the displaceable moiety is displaced from the first surface in the presence of an analyte of interest; and a second surface bearing a capture moiety which specifically binds to the displaceable moiety.

The apparatus defined above may take the form of a relatively cheap, disposable or replaceable component (e.g. as part of a kit) for use in association with other components, such as a "permanent" signal detection means. Alternatively the apparatus may be provided as substantially a "one piece" device, comprising one or more of the following: fluid conducting means; signal detection means for detecting a signal generated in the presence of analyte of interest; and data processing means for processing data output from the signal detection means.

The fluid (preferably liquid) conducting means may perform one or more of the following functions: introduction of the fluid sample to the first surface; transport of any displaced moiety from the first surface to the second surface; and removal of waste fluid through the fluid conducting means. Motive means suitable for use with the fluid conducting means may comprise, for example, a rotary or a peristaltic pump.

Desirably the apparatus is adapted for use with analytes of interest which are suspended or dissolved in liquids (especially body fluids, such as blood, serum, saliva and the like).

The invention will now be further described by way of illustrative example and with reference to the accompanying drawings, in which:
Figures 1 - 3, 6 and 10 are schematic illustrations of assay methods in accordance with the invention;
Figures 4, 5A, 5B, 7, and 11 are traces (arbitrary response units against time) showing SPR signal outputs;
Figures 8 and 12 are graphs of SPR response (arbitrary response units) against time (in seconds); and
Figure 9 is a graph (% displaceable moiety captured at second surface, against flow rate in µl per minute), showing the effect of flow rate on the amount of displaceable moiety captured in one embodiment of the invention, and how at low flow rates 100% of displaceable moiety specifically displaced from the first surface can be captured at the second surface.

### Example 1

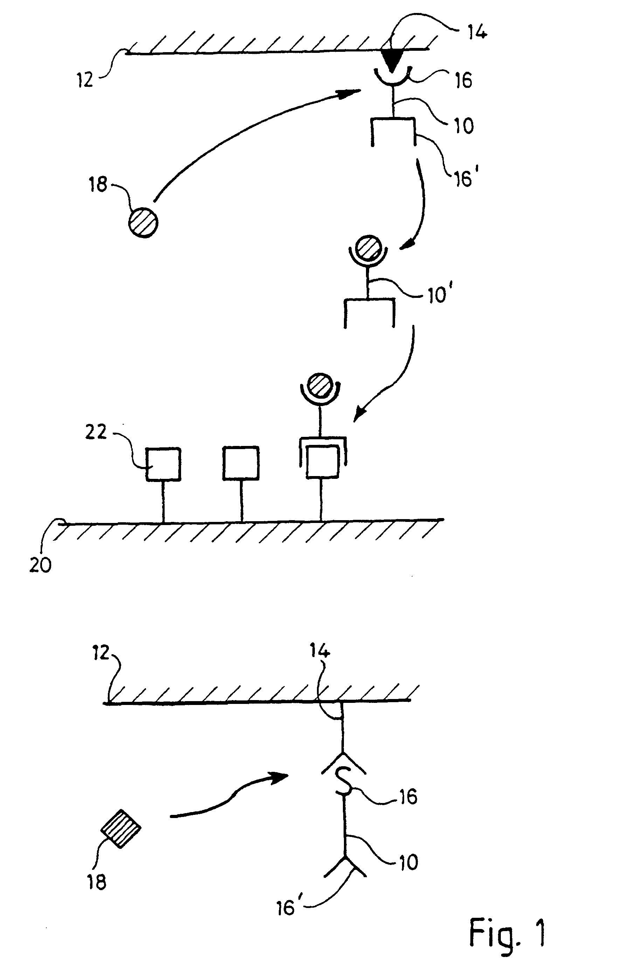

A simple embodiment illustrating the basic concept of the invention is illustrated schematically in Figure 1. In Figure 1, a displaceable moiety is reversibly immobilised at a first surface 12. In the embodiment shown in Figure 1, the displaceable moiety is a bi-specific antibody 10 and the reversible immobilisation is achieved by interaction between the bi-specific antibody 10 and an intervening molecule 14 immobilised, by conventional chemistry, on the first surface 12. For simplicity, only a single molecule 14, and a single specific bispecific antibody 10, are shown. In practice, the first surface 12 would normally be coated with a plurality of intervening molecules 14, and a plurality of bispecific antibody molecules 10 (each having the same binding specificities) would be reversibly immobilised to the surface 12. This reversible immobilisation of the displaceable moiety is such that, under normal assay conditions, and in the absence of the analyte of interest, the displaceable moiety remains attached to the first surface, but is specifically displaced therefrom in the presence of the analyte of interest.

One binding site 16 of the bispecific antibody has binding specificity for the analyte of interest 18. The intervening molecule 14 immobilised on the first surface 12 is a structural analogue of the analyte of interest 18, such that the binding site 16 of the bispecific antibody 10 binds relatively loosely to the molecule 14, thus reversibly immobilising the bispecific antibody 10 on the first surface 12. However, the binding affinity of the binding site 16 for the analogue 14 is much lower (typically 1-10%, preferably 1-5%) than the binding affinity for the analyte of interest 18. Accordingly, upon contacting the first surface 12 with a sample comprising the analyte of interest 18, the analyte 18 competes successfully with analogue 14 for binding to binding site 16, thus specifically displacing the bispecific antibody 10 from the first surface 12. The displaced bispecific antibody 10 is then allowed to contact a second surface 20.

Referring again to Figure 1, the second surface 20 is coated with a plurality of molecules, each of which constitutes a capture moiety 22. The binding site 16 of the bispecific antibody 10 has binding specificity for the capture moiety 22. Accordingly, the bispecific antibody molecule 10 displaced from the first surface is captured at the second surface 20. This capture generates a detectable signal, which is monitored. In the embodiment illustrated in Figure 1, the second surface 20 forms part of an SPR sensor.

Generally, the signal is generated by an interaction between the captured displaceable moiety 10 and the second (capture) surface 20. For example, the second surface 20 may comprise part of an evanescent wave detection device: capture of the displaceable moiety 10 modifies the characteristics of an evanescent wave propagated at the surface 20, in such a way that a detectable signal is produced. Examples of such devices are those which utilise the technique of surface plasmon resonance (SPR) which are now known in the art, or acoustic wave detection systems, wherein the signal is generated by the increase in mass of material bound to the surface 20.

However, other detection systems may also be envisaged, in which a different sort of interaction occurs between the displaceable moiety (when captured) and the second surface. These include the use of electroactive surfaces, wherein the capture of the displaceable moiety by the capture moiety directly modulates the electrochemical properties of the capture moiety, which modulation comprises the detectable signal. Suitable methods and substances are disclosed in our co-pending application filed on even date herewith, under our reference C319/U.

In the embodiment illustrated in Figure 1, the bispecific antibody 10 remains attached to the analyte of interest 18 when it binds to the capture molecule 22 on the second surface 20. In mass-based detection systems (such as SPR devices) this might be an advantage, especially if the displaceable moiety has a very small mass of its own. However, the continued attachment of the analyte 18 to the displaceable moiety 10 might be disadvantageous in other situations, especially where the generation of the detectable signal is not mass-dependent.

### Example 2

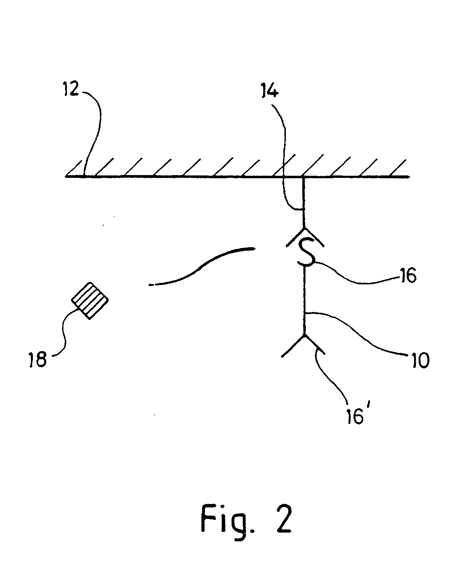

An alternative embodiment (partially illustrated schematically in Figure 2) is also envisaged wherein the nature of the displaceable moiety, and its mode of reversible immobilisation to the first surface, are different to the embodiment illustrated in Figure 1. Functionally comparable components are annotated in Figure 2 using the same reference numerals as in Figure 1.

In Figure 2, there is provided a displaceable moiety 10 reversibly immobilised on a first surface 12, by interaction with an intervening molecule (antibody 14) which is immobilised to the surface 12. Antibody 14 has a specific binding affinity for the analyte of interest 18. The displaceable moiety 10 consists of an antibody molecule fused or covalently coupled to a "mimotope" 16 - a small molecule (normally a peptide, but possibly also a sugar, steroid, nucleic acid or other chemical moiety which may be conjugated to an immunoglobulin) which is a structural analogue of the epitope bound by antibody 14. The mimotope 16 is such that the binding affinity of the antibody 14 for the mimotope 30 is only 1-10% (preferably 1-5%) that of the affinity for the analyte 18. Accordingly, in the presence of the analyte of interest 18, the displaceable moiety 10 is specifically displaced from the first surface 12. The displaceable moiety may then be captured at a second surface (not shown), typically by a capture molecule which recognises the binding site 16' of the antibody portion of the displaceable moiety 10, essentially as indicated in Figure 2. Conveniently the mimotope is a peptide and forms a fusion protein (encoded by a genetic fusion between sequences encoding an immunoglobulin and the peptide). Alternatively the mimotope may be joined to an immunoglobulin molecule (or antigen binding portion thereof) by any one of conventional chemical techniques (see, for example, "Bioconjugate Techniques", Hermanson, Academic Press, 1996).

An advantage of the embodiment illustrated in Figure 2 is that the displaceable moiety 10 when displaced from the first surface 12, consists in large part of a conventional antibody molecule and is not attached to the analyte of interest 18. The presence of the analyte of interest 18 at the second surface (as in the embodiment illustrated in Figure 1) may sometimes interfere, in some embodiments, with signal generation.

### Example 3

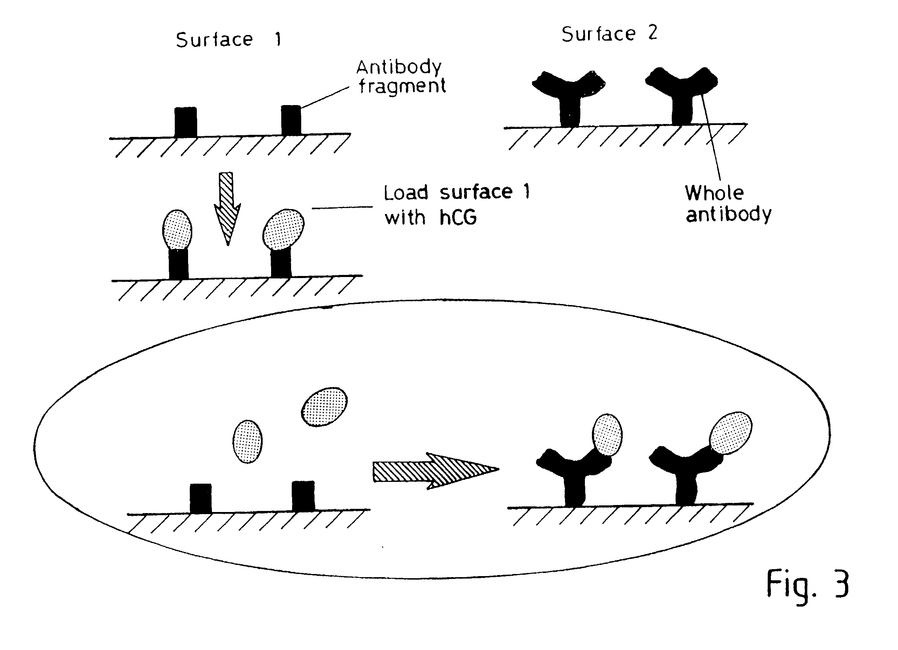

This example relates to the dissociation of human chorionic gonadotrophin (hCG) from an immobilised antibody fragment located at one surface and capture/detection by antibody at a second surface. Figure 3 schematically illustrates this detection principle. This example is performed using the Biacore AB Bialite ™ fitted with an upgrade kit and employs the use of surface plasmon resonance.

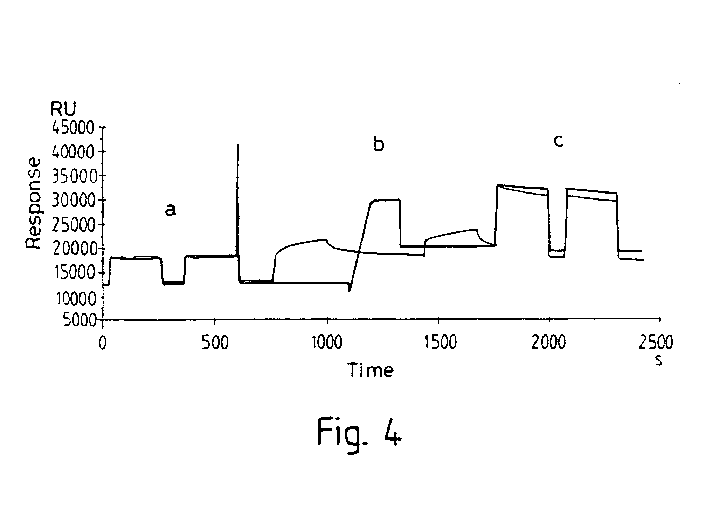

Initially, a CM5 sensor chip was set up as follows:
i. A new CM5 sensor chip was docked in the Bialite™ biosensor and equilibrated with HBS (HEPES buffered saline) running buffer. The temperature was maintained at 25°C and the flow rate set to 10 µl/min.
ii. The liquid flow was set to cross two flow cells, flow cell 1 "first surface" and flow cell 2 "second surface" in series. The dextran surface was activated using 1-ethyl(dimethylaminopropyl) carbodiimide (EDC) and N-hydroxysuccinimide (NHS) activating chemicals from the Biacore AB amine coupling kit by injecting the EDC/NHS mixture into the sample loop and loading 40 µl. The activation was repeated and can be seen marked (a) in figure 4. The Bialite™ response at the first surface is shown as a thin line, whereas the response at the second surface is shown as a thick line. This line thickness identification is used throughout subsequent Bialite™ traces.
iii. Following activation, 40 µl of a 50 µg/ml solution of single chain antibody fragment recognising hCG (scFv3299HisH2) was injected across flow cell 1. This was followed by injection of 40 µl of a 50µg/ml solution of a whole antibody recognising hCG (3299) across flow cell 2 and a further injection of 40 µl of a 50 µg/ml solution of scFv3299HisH2 across flow cell 1. These coupling steps can be seen marked (b) in figure 4.
iv. Unreacted sites on the CM5 sensor chip were blocked by two sequential injections of 40 µl of a 1M solution of ethanolamine. These can be seen marked (c) in figure 4.

By way of explanation, the sequence of scFv3299 is as disclosed in WO96/27612 (wherein it is described as FvKC-II), but with the *Bst*EII*-Sac*I DNA linker fragment replaced with a *Bst*EII-*Sac*I fragment encoding a flexible linker yielding the sequence: The DNA sequence encoding the linker is described by Jackson *et al* ("Selection of variants of antibodies and other protein molecules using display on the surface of bateriophage fd" in Protein Engineering: A Practical Approach, eds. Rees, Sternberg and Wetzel, publ. IRL Press, Oxford, 1992). It will be appreciated that numerous other anti-hCG antibodies would be equally suitable. Others available are listed in Linscott's Directory of Immunological and Biological Reagents (9th edition, 1996-7) and include, for example, an anti α-hCG monoclonal available from Biostride Inc. (CA, USA), and an anti β-hCG monoclonal available from Biogenesis Ltd (Poole, Dorset, UK).

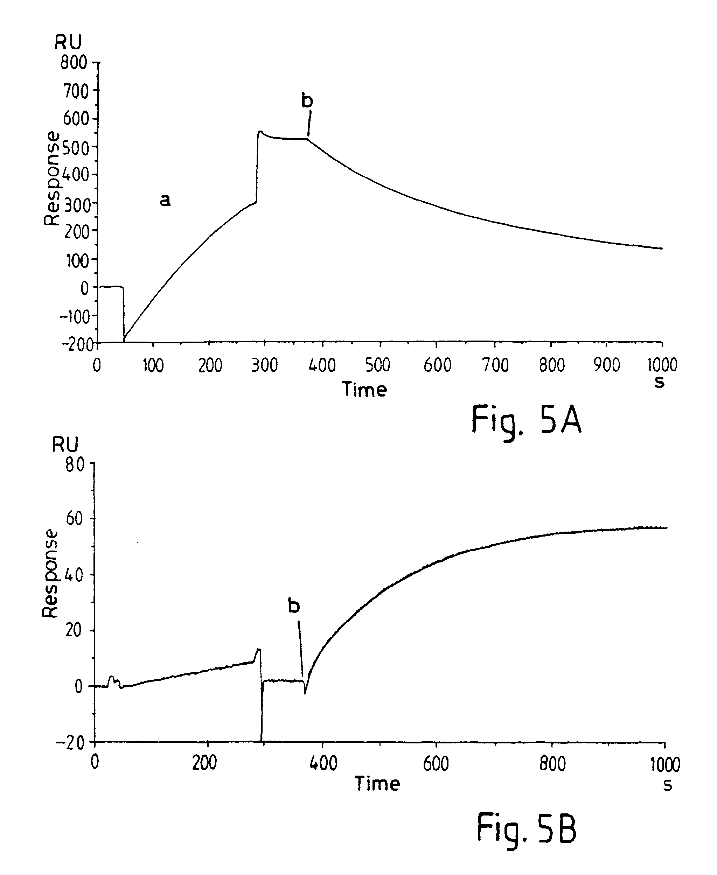

The capture/detection of hCG takes place as follows:
v. 200 µl of hCG (10 IU/ml) was incubated with 20 µl of a second monoclonal antibody recognising hCG (termed 3468, at 100 µg/ml) for 30 minutes at room temperature (again, antibodies other than 3468 would be suitable). This was performed in order to increase the effective mass of hCG and thus increase the sensitivity of detection.
vi. 40 µl of the premixed hCG-3468 solution was injected across flow cell 1. This can be seen marked (a) in figure 5A.
vii. Immediately following the injection, the buffer flow was altered to pass over the second surface after leaving the first surface. The point at which this took place is marked (b) in figure 5B.

Figure 5A shows that hCG-3468 complex dissociating from the first surface can be bound and detected at the second surface by capture with anti-hCG antibody 3299 (Fig. 5B). Those skilled in the art will appreciate that, in practice, there is no need for the first surface to generate a detectable signal - such was the arrangement in this example for the purposes of investigation only. In a normal arrangement only the capture (second) surface would give rise to a detectable signal, such that the first surface could be any suitable solid support. Moreover, in the assay method of the invention, conditions would be arranged such that hCG was displaced from surface 1 only in the presence of the analyte of interest. The example nevertheless demonstrates the principle of the invention.

### Example 4

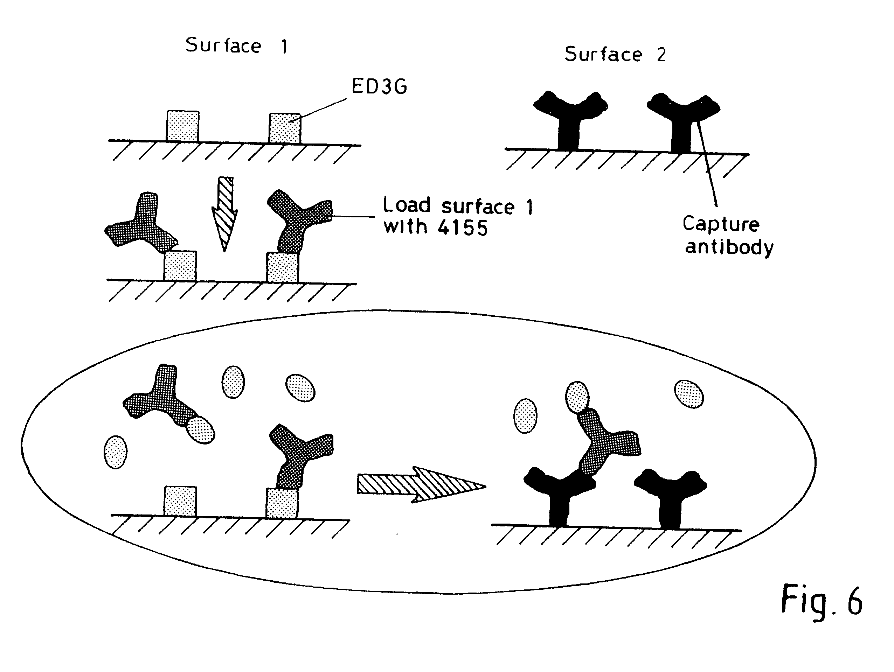

This example relates to the specific displacement of an antibody (4155) recognising estrone 3-glucuronide (E3G) from a low affinity E3G analogue (estradiol 3-glucuronide [ED3G]) at one surface and capture and detection by an antibody recognising immunoglobulin at a second surface. Figure 6 schematically illustrates this detection principle.

The 4155 monoclonal cell line was prepared and screened according to the methods described by Gani *et al.,* (1994 J. Steroid Biochem. Molec. Biol. 48, 277-282). The Gani *et al.* publication relates to development of anti-progesterone antibodies, but essentially identical techniques were employed in producing antibodies reacting with estrone and analogues thereof. Antibodies other than that obtainable from cell line 4155 may readily be produced by those skilled in the art (using such techniques): such antibodies would have qualitatively similar properties. Moreover, a commercially available anti-estrone glucuronide monoclonal antibody (from Wallaceville Animal Research Centre, New Zealand) is described in Linscott's Directory of Immunological and Biological Reagents (9th edition, 1996-7).

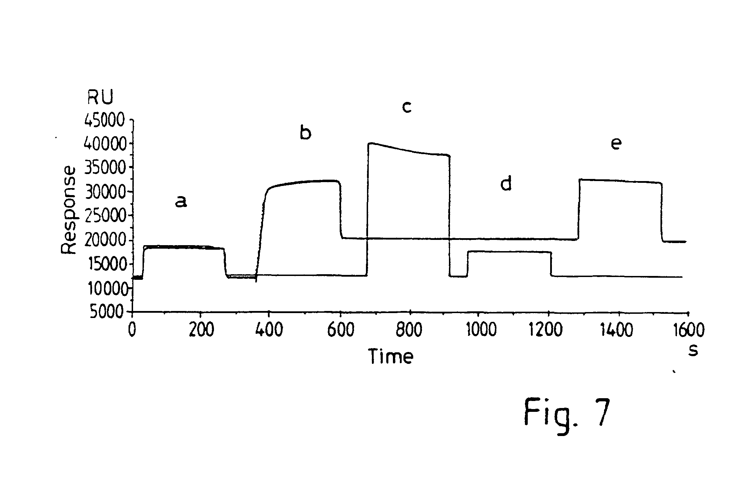

Initially, a CM5 sensor chip was set up as follows:
i. A new CM5 sensor chip was docked in the Bialite™ biosensor and equilibrated with HBS running buffer. The temperature was maintained at 25°C and the flow rate set to 10 µl/min.
ii. The liquid flow was set to cross a first surface ("surface 1") and a second surface ("surface 2") in series. The dextran surface was activated using EDC and NHS activating chemicals from the Biacore AB amine coupling kit by injecting the EDC/NHS mixture into the sample loop and loading 40 µl. This can be seen marked (a) on figure 7.
iii. The buffer flow was set to cross only surface 2 and 40 µl of rabbit anti-mouse IgG in sodium acetate buffer, pH 5.0 was injected. This can be seen marked (b) on figure 7.
iv. Following IgG coupling to surface 2 the buffer flow was changed to flow only over surface 1 and 40 µl ethylene diamine (EDA) (20% v/v) injected. This can be seen marked (c) on figure 7.
v. 20 µl of ED3G (5 mg/ml) was added to 80 µl of a solution of NHS/EDC activation solution, mixed and incubated for 7 minutes at room temperature. 40 µl of this preincubated ED3G solution was then injected across surface 1 (this can be seen marked (d) on figure 7).
vi. The buffer flow was set to flow only over surface 2 and 40 µl of a 1M solution of ethanolamine was injected. This can be seen marked (e) on figure 7.

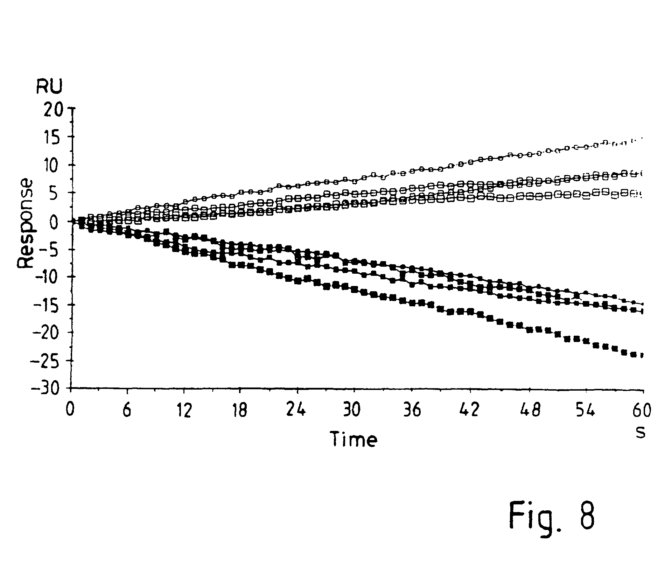

Displacement and recapture of 4155 was performed as follows:
vii. 4155 was loaded onto surface 1 by the injection of 20 µl of a 100 µg/ml solution of 4155.
viii. Specific displacement from surface 1 and recapture/detection at surface 2 was induced by the injection of a 1 mg/ml solution of E3G across surfaces 1 and 2 for two minutes at various flow rates. The results of this can be seen in figure 8. in which the filled boxes show the specific displacement of 4155 from surface 1 by E3G and the open boxes show the capture of 4155 at surface 2. Increasing box size denotes an increase in flow rate where the smallest size box represents 2 µl/min and subsequent box sizes are of data collected at 5, 10 and 20 µl/min.

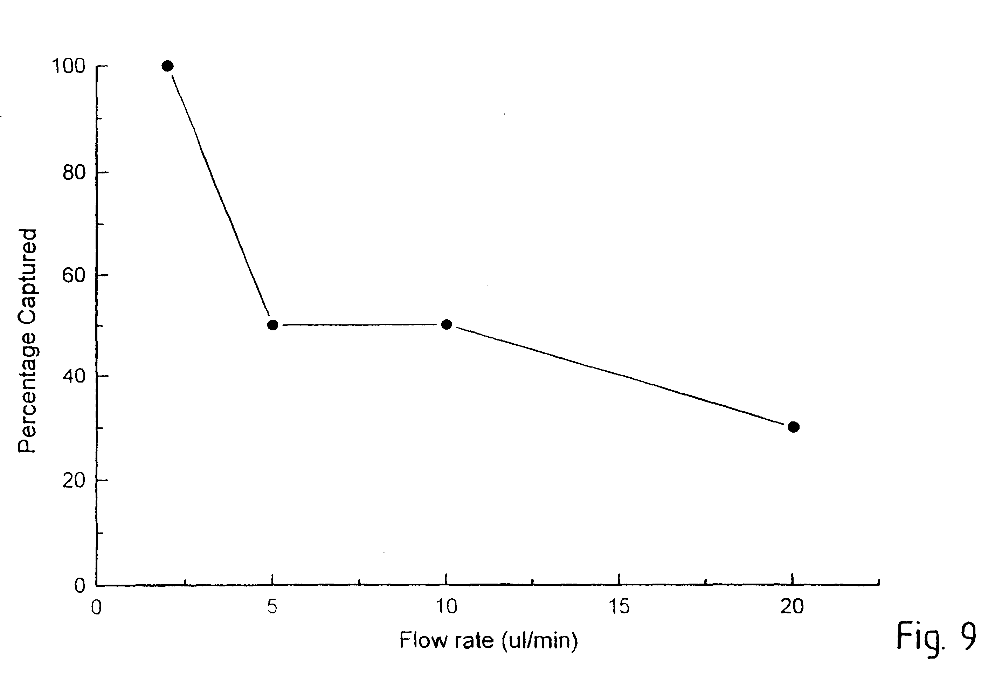

The percentage of the displaced 4155 captured and detected at surface 2 is clearly related to the flow rate (see figure 9). An increase in flow rate decreases the percentage of displaced material. Low flow rates (less than 5µl/min) can ensure all the displaced material is captured.

This example shows how analyte (E3G) can be assayed by inducing the specific displacement of a molecule reversibly bound to one surface and subsequent recapture of the displaced material at a second distinct surface by, for example, an antibody recognising the displaced molecule.

### Example 5

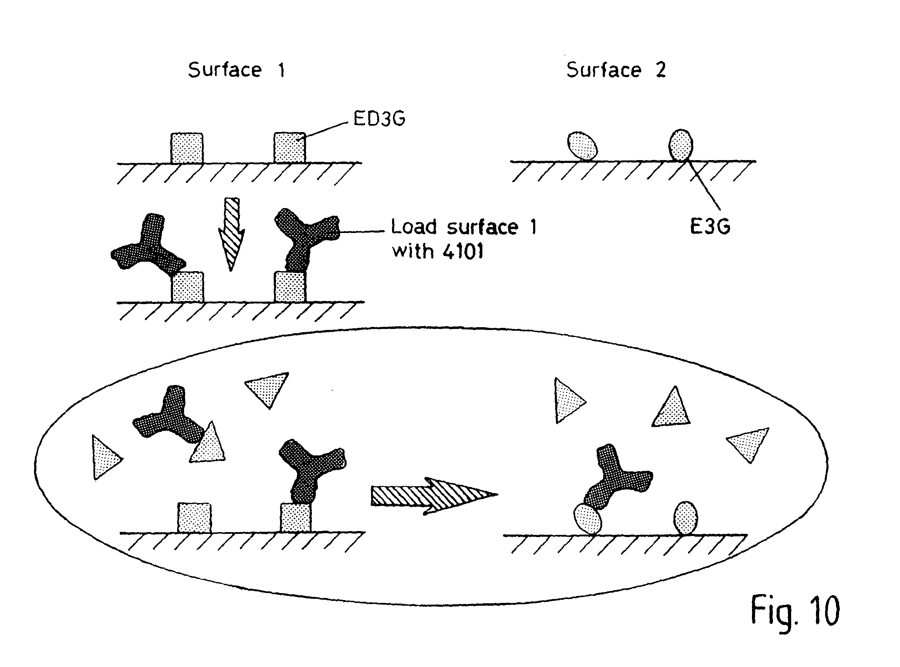

This example relates to the specific displacement of an antibody (4101) recognising estrone 3-glucuronide (E3G) from a low affinity E3G analogue (estradiol 3-glucuronide [ED3G]) at one surface and capture by E3G at a second, detecting surface. Figure 10 schematically illustrates this detection principle.

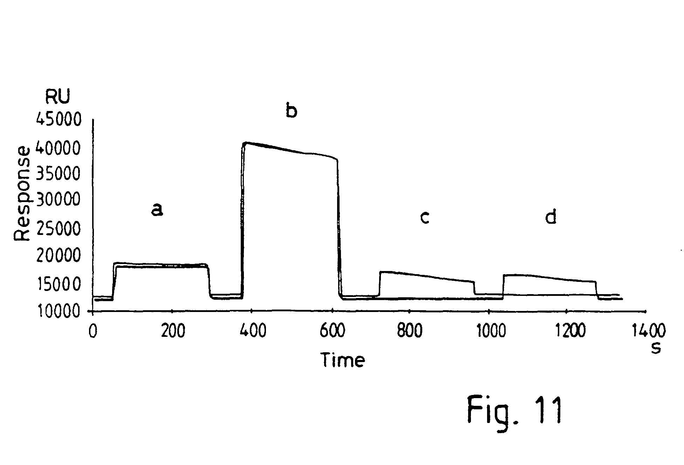

Initially, a CM5 sensor chip was set up as follows:
i. A new CM5 sensor chip was docked in the Bialite™ biosensor and equilibrated with HBS running buffer. The temperature was maintained at 25 °C and the flow rate set to 10 µl/min.
ii. The liquid flow was set to cross a first surface ("surface 1") and a second surface ("surface 2") in series. The dextran surface was activated using EDC and NHS activating chemicals from the Biacore AB amine coupling kit by injecting the EDC/NHS mixture into the sample loop and loading 40 µl. This can be seen marked (a) on figure 11.
iii. 40 µl of EDA (20% v/v) was injected across surface 1 and 2. This can be seen marked (b) on figure 11.
iv. 20 µl of ED3G (5 mg/ml) was added to 80 µl of a solution of NHS/EDC activation solution, mixed and incubated for 7 minutes at room temperature. 40 µl of this preincubated ED3G solution was then injected across surface 1 (this can be seen marked (c) on figure 11).
v. 20 µl of E3G (5 mg/ml) was added to 80 µl of a solution of NHS/EDC activation solution, mixed and incubated for 7 minutes at room temperature. 40 µl of this preincubated E3G solution was then injected across surface 2 (this can be seen marked (d) on figure 11).

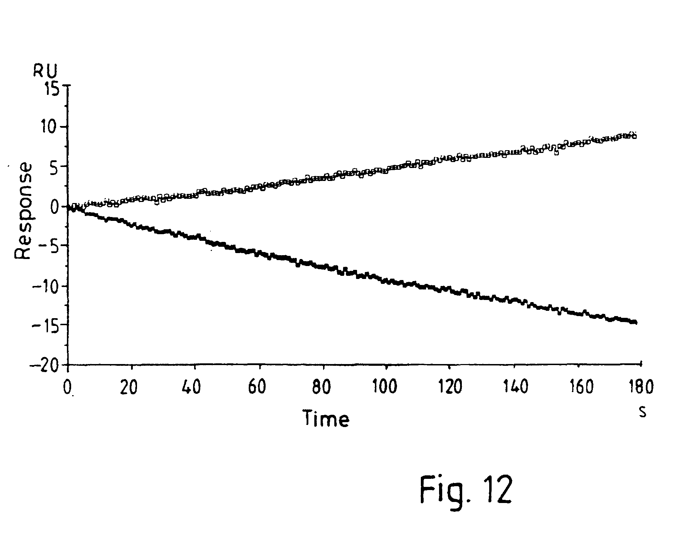

Specific displacement and capture of 4101 was performed as follows:
vi. 4101 was loaded onto surface 1 by the injection of 20 µl of a 100 µg/ml solution of 4101 across surface 1.
vii. Displacement of 4101 from surface 1 and recapture/detection at surface 2 was induced by the injection of 20 µl of a 1 mg/ml solution of estrone 3-sulphate [E3S] across surface 1 and 2. The displacement from surface 1 and capture/detection at surface 2 can be seen in figure 12.

In this example E3S represents the analyte. This example shows how analyte can be assayed by inducing the displacement of a molecule reversibly bound to a lower affinity analogue of the analyte (ED3G) at one surface and subsequently recaptured and detected at a second distinct surface by binding to a higher affinity analogue of the analyte (E3G).

### Example 6

This example shows the production of a colour change brought about by the displacement/capture of a glucose, oxidase/antibody conjugate in response to analyte addition. The displaced glucose oxidase/antibody conjugate is captured on a thin layer of starch impregnated with KI, horse radish peroxidase and a capture molecule. The captured glucose oxidase/antibody conjugate utilises glucose to generate H₂O₂, which oxidises iodide to iodine in the presence of peroxidase. The iodine generated then reacts with starch, resulting in a colour change from brown to blue.

### Two surfaces are set up as follows:

### First Surface ("Surface 1")

i. An estradiol 3-glucuronide (ED3G)/ovalbumin conjugate is prepared by resuspending 2.6 mg ED3G in 2 ml of a freshly prepared solution of EDC (0.1 M) and NHS (0.02 M) and incubating for 15 min at room temperature.
ii. To the ED3G solution, 2 ml ovalbumin (10 mg/ml) is added and this is incubated for 2.5 h at room temperature with constant mixing.
iii. The conjugated ED3G/ovalbumin solution is then dialysed for 16 h against 1L phosphate buffered saline containing 0.1 % sodium azide ("PBSA").
iv. Griener HB wells are coated with the dialysed ED3G-ovalbumin conjugate by incubating 100 µl ED3G-ovalbumin (diluted 1 in 10 with PBSA) in the wells for 2 h at room temperature.
v. A conjugate of glucose oxidase and monoclonal antibody 4155 is prepared by mixing 1ml glucose oxidase in 100 mM phosphate buffer pH 6.5 with 1 ml of monoclonal antibody 4155 (also in 100 mM phosphate buffer pH 6.5) in a glass tube at a protein concentration of 5 mg/ml. To this 220 µl of 0.02% glutaraldehyde is added dropwise with constant mixing. The tube is wrapped in aluminium foil and mixed end over end for 30 min at room temperature. 250 µl of 1 M ethanolamine is added and mixing continued for a further 30 min. The solution is then dialysed against 100 mM PBS at room temperature for 24 h then centrifuged at 10,000 rpm for 30 min at 4°C.
vi. The Griener HB surface is then primed by incubating 100 µl glucose oxidase-4155 conjugate per well for I h at room temperature. Wells are then rinsed with PBS to remove unbound glucose oxidase/4155 conjugate. Wells are stored containing 100 µl PBS at 4°C until required.

### Second Surface ("Surface 2")

i. A 200 µl solution containing 100 µl estrone 3-glucuronide (E3G)-ovalbumin conjugate (prepared as described for the ED3G-ovalbumin conjugate), 5% (w/v) starch, 10 mg/ml horseradish peroxidase and 0.1 M KI in PBS is incubated in a Griener HB well for 16 h at room temperature. The surface is brown in colour.

Analyte (1 ml estrone 3 glucuronide at 0.1 mM) in a 10 mM glucose solution in PBS is pumped at 0.2 ml/min into the well with the primed surface 1 and at the same time solution from this well is pumped at 0.2 ml/min into the well containing surface 2. In order that the well with surface 2 does not overflow, solution is also pumped out of the well at 0.2 ml/min.

Upon starting the flow of test solution from surface 1 to surface 2 the colour of surface 2 changes from brown to blue as the iodide is oxidised to iodine by the H₂O₂ generated by the oxidation of glucose by glucose oxidase.

### Example 7

This example relates to the modulation of fluorescence intensity at a surface in response to analyte. Upon challenge with analyte, molecules are displaced from a first surface. These displaced molecules are then able to modulate the intensity of fluorescence generated at a second surface.

Two surfaces are set up as follows:

### First Surface ("Surface 1")

i. An estradiol 3-glucuronide (ED3G)/ovalbumin conjugate is prepared by resuspending 2.6 mg ED3G in 2 ml of a freshly prepared solution of EDC (0.1 M) and NHS (0.02 M) and incubating for 15 min at room temperature.
ii. To the ED3G solution 2 ml ovalbumin (10 mg/ml) is added and this is incubated for 2.5 h at room temperature with constant mixing.
iii. The conjugated ED3G/ovalbumin solution is then dialysed for 16 h against 1L PBSA.
iv. Griener HB wells are coated with the dialysed ED3G/ovalbumin conjugate by incubating 100 µl ED3G/ovalbumin (diluted 1 in 10 with PBSA) in the wells for 2 h at room temperature.
v. A conjugate of cellulose binding domain (CBD)/monoclonal antibody 4155 is prepared by mixing 1 ml CBD in 100 mM phosphate buffer pH 6.5 with 1 ml of monoclonal antibody 4155 (also in 100 mM phosphate buffer pH 6.5) in a glass tube at a protein concentration of 5 mg/ml. To this 220 µl of 0.02% glutaraldehyde is added dropwise with constant mixing. The tube is wrapped in aluminium foil and mixed end over end for 30 min at room temperature. 250 µl of 1 M ethanolamine is added mixing continued for a further 30 min. The solution is then dialysed against 100 mM PBS at room temperature for 24 h then centrifuged at 10,000 rpm for 30 min at 4°C. The supernatant is stored at 4°C until required.
vi. The Griener HB surface is then primed by incubating 100 µl CBD-4155 conjugate per well for 1 h at room temperature. Wells are then rinsed with PBSA to remove unbound CBD/4155 conjugate.

### Second Surface ("Surface 2")

i. A 1 ml solution of H₃PO₄ swollen Avicel (0.2 %) is incubated with 50 µl locust bean gum (LBG)-FITC conjugate for 1h with constant mixing at room temperature.
ii. LBG-FITC A vicel is washed by subjecting the particles to centrifugation at 1300 rpm for 10 min at room temperature and resuspending the pellet in PBS. This wash procedure is repeated a second time.
iii. Following the second wash the LBG-FITC loaded particles are resuspended in 50 µl PBS and stored at 4°C until required.

Analyte (100 µl E3G at 1 mg/ml) is incubated for 1 h in primed Griener HB wells (surface 1) at room temperature. The incubated solution is then removed from the wells and added to LBG-FITC loaded particles. A decrease in fluorescence intensity due to the displacement of LBG-FITC by CDB-4155 on the Avicel particles is monitored using a fluorescent activated cell sorter, or by a cell sorter (e.g. the Coulter Elite™ cell sorter).

### Example 8

This example shows the production of an electrical change brought about by the displacement/capture of a bispecific antibody fragment construct in response to analyte addition. It relates to the specific displacement of the first antibody binding site (recognising estrone-3-glucuronide (E3G) and derived from mouse monoclonal antibody 4155) from a low affinity E3G analogue (estradiol 3-glucuronide (ED3G)) from a first surface, and capture and detection through the second antibody binding site recognising an electroactive molecule immobilised at a second surface. The second surface is a gold electrode coated with a monolayer of the electroactive molecule. The electroactive molecule is a carbazole residue, coupled to a hexyl pendant portion, which is in turn coupled to the gold surface by a thiol group. Our co-pending European Patent application, filed on even date herewith, describes how the binding of selected antibodies can alter (modulate) the electrical current flowing through the electrode under the influence of an applied voltage. Here the binding of the bispecific antibody fragment to the electroactive molecule thus modulated the current flow in a manner which was directly related to the concentration of analyte originally introduced.

The 4155 monoclonal cell line was prepared and screened according to methods described by Gani *et al.* (1994 J. Steroid Biochem. Molec. Biol. **48.** 277-282). The Gani *et al* publication relates to the development of anti-progesterone antibodies, but essentially identical techniques were employed in producing antibodies reacting with estrone and analogues thereof. Antibodies other than that obtainable from cell line 4155 may readily be produced by those skilled in the art (using such techniques): such antibodies would have qualitatively similar properties. Monoclonal antibodies against the electroactive carbazole molecule, another hapten, can also be produced using these methods. The cloning of the genes for the two antibody binding specificities and their use in the construction of a bispecific antibody fragment can easily be accomplished by methods well known to those skilled in the art; (see for example WO 97/14719).

Two surfaces are set up as follows:

### First surface ("Surface 1")

i. An estradiol 3-glucuronide (ED3G)/ovalbumin conjugate is prepared by resuspending 2.6mg of ED3G in 2ml freshly prepared solution of EDC (0.1M) and NHS (0.02M) and incubating for 15 mins at room temperature.
ii. To the ED3G solution, 2ml ovalbumin (10mg/ml) is added and this is incubated for 2.5hr at room temperature with constant mixing.
iii. The conjugated ED3G/ovalbumin solution is then dialysed for 16hr against 1L phosphate buffered saline containing 0.1% sodium azide ("PBSA").
iv. Greiner HB wells are coated with dialysed ED3G-ovalbumin conjugate by incubating 100 µl ED3G-ovalbumin (diluted 1 in 10 with PBSA) in the weils for 2 hr at room temperature.
v. The Greiner HB-ED3G-ovalbumin surface is then primed by incubating 100 µl of a solution of the bispecific antibody fragment (100µg/ml in PBSA) per well for 1 hr at room temperature. Wells are then rinsed with PBSA to remove unbound bispecific antibody fragment. Wells were stored containing 100µl of PBSA at 4°C until required.

### Second surface ("Surface 2")

i. The thiol terminus alkyl pendant electroactive (carbazole) molecule was dissolved in nitrogen purged ethanol or DMF solution.
ii. A clean pure gold surface was dipped into the solution containing the electroactive moiety for 24hr in the dark.
iii. The electroactive monolayer on the gold surface was washed in a suitable solvent (e.g. dichloromethane) to remove any unbound molecules and then stored overnight in a suitable solvent that has been nitrogen purged.
iv. The electrode was dried in a stream of nitrogen to remove solvent from the electrode surface.
v. The electrode was transferred through a series of increasingly dilute acetonitrile: water mixes to improve the monolayer's molecular orientation, which results in a monolayer which is stable in an aqueous environment.

### Assay

Electrochemical measurements are performed by cyclic voltammetry or chronoamperometry on a Princeton Applied Research Corporation Scanning Potentiostat (model 362) together with a Bryan flat bed X-Y recorder (model A25000) and a EH&G model 273A Princeton Applied Research Potentiostat/Galvanostat (using Echem and Lotus 123 to process the data).
(a) The background electrochemistry is read and stored once a stable scan had been produced.
(b) The sample to be read is added to surface 1 (the well) and incubated for 15 minutes at room temperature.
(c) The sample is removed from the well and added to surface 2 (the electrode surface), and again incubated at room temperature for 15 minutes.
(d) The electrode surface is washed with MilliQ water and the electrochemistry read using the same conditions as (b) above. The modulation of the current flow gives a measure of the analyte concentration which may be expressed in molar concentration units if the system has been calibrated against standard E3G samples.

## Claims

1. A method of detecting the presence of an analyte of interest in a sample, the method comprising the steps of: providing a first surface having reversibly immobilised thereon a displaceable moiety; exposing the first surface to a sample comprising the analyte of interest, the analyte of interest specifically displacing the displaceable moiety from the first surface in an affinity-related manner; causing the displaceable moiety displaced from the first surface to contact a second surface bearing a capture moiety which specifically binds to the displaceable moiety, so as to capture the displaceable moiety on the second surface, said capture generating a detectable signal; and detecting the signal; wherein said detection is performed by means other than SPR, and wherein the displaceable moiety cannot generate the signal which is detected in the assay unless and until the displaceable moiety is captured on the second surface.

2. A method according to claim 1, wherein the displaceable moiety comprises an immunoglobulin molecule or an antigen-binding derivative thereof.

3. A method according to claim 1 or 2, wherein the displaceable moiety comprises a bispecific antibody or bispecific antigen-binding antibody derivatives.

4. A method according to any one of claims 1, 2 or 3, wherein the displaceable moiety comprises a portion which facilitates reversible immobilisation on the first surface.

5. A method according to any one of the preceding claims, wherein the displaceable moiety comprises a fusion protein.

6. A method according to any one of the preceding claims, wherein the displaceable moiety comprises a mimotope which is an analogue of the analyte of interest.

7. A method according to any one of the preceding claims, wherein the first surface comprises a plurality of intervening molecules which bind relatively loosely to the displaceable moiety, such that the binding affinity of the intervening moiety for the analyte of interest is greater than that of the displaceable moiety for the intervening moiety.

8. A method according to claim 7, wherein the intervening molecule is an analogue (e. g. mimotope) of the analyte of interest.

9. A method according to any one of the preceding claims, wherein the capture moiety comprises an immunoglobulin molecule or an antigen-binding variant thereof.

10. A method according to any one of the preceding claims, wherein the displaceable moiety and the capture moiety comprise the members of a specific binding pair.

11. A method according to any one of the preceding claims, wherein the detectable signal comprises the generation of, or the modulation of, an evanescent or acoustic wave.

12. A method according to any one of claims 1-10, wherein capture of the displaceable moiety by the capture moiety directly modulates the electrochemical properties of the capture moiety, which modulation comprises the detectable signal.

13. A method according to any one of the preceding claims, wherein the first and second surfaces are provided on separate respective first and second supports.

14. A method according to any one of claims 1-12, wherein the first and second surfaces are provided on a single support.

15. A method according to any one of the preceding claims, wherein the first and/or second surface is provided on a solid support which is planar, particulate or porous.

16. A method according to any one of the preceding claims, wherein the analyte of interest is selected from the group consisting of: steroid hormones, protein hormones, nucleic acids, peptides, bacterial or viral antigens, and immunoglobulins.

## Patentansprüche

1. Verfahren zum Detektieren des Vorliegens eines interessierenden Analyten in einer Probe, wobei das Verfahren die folgenden Schritte umfasst:
- Bereitstellen einer ersten Oberfläche, die eine verdrängbare Gruppierung daran reversibel immobilisiert hat;
- Aussetzen der ersten Oberfläche einer Probe, die den interessierenden Analyten umfasst, wobei der interessierende Analyt die verdrängbare Gruppierung in einer Affinitäts-bezogenen Art spezifisch von der ersten Oberfläche verdrängt,
- Bewirken, dass die verdrängbare Gruppierung, die von der ersten Oberfläche verdrängt wurde, mit einer zweiten Oberfläche in Kontakt kommt, die eine Abfanggruppierung trägt, die spezifisch an die verdrängbare Gruppierung bindet, so dass die verdrängbare Gruppierung an der zweiten Oberfläche abgefangen wird, wobei das Abfangen ein detektierbares Signal erzeugt, und
- Detektieren des Signals, wobei die Detektion durch andere Mittel als SPR erfolgt und wobei die verdrängbare Gruppierung das Signal, das im Assay detektiert wird, nicht erzeugen kann, wenn die verdrängbare Gruppierung nicht an der zweiten Oberfläche abgefangen ist oder bis die verdrängbare Gruppierung an der zweiten Oberfläche abgefangen ist.

2. Verfahren nach Anspruch 1, wobei die verdrängbare Gruppierung ein Immunglobulin-Molekül oder ein Antigen-bindendes Derivat davon umfasst.

3. Verfahren nach Anspruch 1 oder 2, wobei die verdrängbare Gruppierung einen bispezifischen Antikörper oder bispezifische Antigen-bindende Antikörperderivate umfasst.

4. Verfahren nach mindestens einem der Ansprüche 1, 2 oder 3, wobei die verdrängbare Gruppierung einen Teil umfasst, der eine reversible Immobilisierung an der ersten Oberfläche erleichtert.

5. Verfahren nach mindestens einem der vorangehenden Ansprüche, wobei die verdrängbare Gruppierung ein Fusionsprotein umfasst.

6. Verfahren nach mindestens einem der vorangehenden Ansprüche, wobei die verdrängbare Gruppierung ein Mimotop umfasst, welches ein Analogon des interessierenden Analyten ist.

7. Verfahren nach mindestens einem der vorangehenden Ansprüche, wobei die erste Oberfläche eine Vielzahl dazwischen liegender Moleküle umfasst, die relativ lose an die verdrängbare Gruppierung binden, so dass die Bindungsaffinität der dazwischen liegenden Gruppierung für den interessierenden Analyten größer als die der verdrängbaren Gruppierung für die dazwischen liegende Gruppierung ist.

8. Verfahren nach Anspruch 7, wobei das dazwischen liegende Molekül ein Analogon (z.B. Mimotop) des interessierenden Analyten ist.

9. Verfahren nach mindestens einem der vorangehenden Ansprüche, wobei die Abfanggruppierung ein Immunglobulin-Molekül oder eine Antigen-bindende Variante davon umfasst.

10. Verfahren nach mindestens einem der vorangehenden Ansprüche, wobei die verdrängbare Gruppierung und die Abfanggruppierung die Glieder eines spezifischen Bindungspaares umfassen.

11. Verfahren nach mindestens einem der vorangehenden Ansprüche, wobei das detektierbare Signal die Erzeugung oder die Modulation einer abklingenden oder akustischen Welle umfasst.

12. Verfahren nach mindestens einem der Ansprüche 1 bis 10, wobei das Abfangen der verdrängbaren Gruppierung durch eine Abfanggruppierung die elektrochemischen Eigenschaften der Abfanggruppierung direkt moduliert, wobei die Modulation ein detektierbares Signal umfasst.

13. Verfahren nach mindestens einem der vorangehenden Ansprüche, wobei die erste und die zweite Oberfläche an getrennten entsprechenden ersten und zweiten Trägern bereitgestellt werden.

14. Verfahren nach mindestens einem der Ansprüche 1 bis 12, wobei die erste und die zweite Oberfläche an einem einzelnen Träger bereitgestellt werden.

15. Verfahren nach mindestens einem der vorangehenden Ansprüche, wobei die erste und/oder zweite Oberfläche an einem festen Träger bereitgestellt sind/ist, welcher planar, partikelförmig oder porös ist.

16. Verfahren nach mindestens einem der vorangehenden Ansprüche, wobei der interessierende Analyt aus der Gruppe, bestehend aus Steroidhormonen, Proteinhormonen, Nukleinsäuren, Peptiden, bakteriellen oder viralen Antigenen und Immunglobulinen, ausgewählt wird.

## Revendications

1. Procédé de détection de la présence d'un analyte d'intérêt dans un échantillon, le procédé comprenant les étapes consistant à : fournir une première surface ayant une fraction déplaçable immobilisée de manière réversible sur celle-ci ; exposer la première surface à un échantillon comprenant l'analyte d'intérêt, l'analyte d'intérêt déplaçant spécifiquement la fraction déplaçable à partir de la première surface en fonction de leur affinité ; faire que la fraction déplaçable, déplacée à partir de la première surface, soit en contact avec une deuxième surface renfermant une fraction de capture, qui se lie spécifiquement à la fraction déplaçable afin de capturer la fraction déplaçable sur la deuxième surface, ladite capture générant un signal détectable ; et détecter le signal ; procédé dans lequel ladite détection est effectuée par des moyens autres que la R.P.S. et dans lequel la fraction déplaçable ne peut pas générer le signal, qui est détecté dans le dosage, à moins que ou jusqu'à ce que la fraction déplaçable soit capturée sur la deuxième surface.

2. Procédé selon la revendication 1, dans lequel la fraction déplaçable comprend une molécule d'immunoglobuline ou un dérivé de celle-ci se liant à un antigène.

3. Procédé selon la revendication 1 ou 2, dans lequel la fraction déplaçable comprend un anticorps bi-spécifique ou des dérivés d'anticorps bi-spécifiques se liant à un antigène.

4. Procédé selon l'une quelconque des revendications 1, 2 ou 3, dans lequel la fraction déplaçable comprend une partie qui facilite une immobilisation réversible sur la première surface.

5. Procédé selon l'une quelconque des revendications précédentes, dans lequel la fraction déplaçable comprend une protéine de fusion.

6. Procédé selon l'une quelconque des revendications précédentes, dans lequel la fraction déplaçable comprend un mimotope, qui est un analogue de l'analyte d'intérêt.

7. Procédé selon l'une quelconque des revendications précédentes, dans lequel la première surface comprend une multiplicité de molécules intercalaires qui se lient, de manière relativement relâchée, à la fraction déplaçable de sorte que l'affinité de liaison de la fraction intercalaire pour l'analyte d'intérêt soit supérieure à celle de la fraction déplaçable pour la fraction intercalaire.

8. Procédé selon la revendication 7, dans lequel la molécule intercalaire est un analogue (à titre d'exemple un mimotope) de l'analyte d'intérêt.

9. Procédé selon l'une quelconque des revendications précédentes, dans lequel la fraction de capture comprend une molécule d'immunoglobuline ou un variant de celle-ci se liant à un antigène.

10. Procédé selon l'une quelconque des revendications précédentes, dans lequel la fraction déplaçable et la fraction de capture constituent les membres d'une paire de liaison spécifique.

11. Procédé selon l'une quelconque des revendications précédentes, dans lequel le signal détectable comprend la génération ou la modulation d'une onde évanescente ou sonore.

12. Procédé selon l'une quelconque des revendications 1 à 10, dans lequel la capture de la fraction déplaçable par la fraction de capture module directement les propriétés électrochimiques de la fraction de capture, laquelle modulation comprend le signal détectable.

13. Procédé selon l'une quelconque des revendications précédentes, dans lequel les première et deuxième surfaces sont fournies sur des premier et deuxième supports respectifs séparés.

14. Procédé selon l'une quelconque des revendications 1 à 12, dans lequel les première et deuxième surfaces sont fournies sur un seul support.

15. Procédé selon l'une quelconque des revendications précédentes, dans lequel la première et/ou la deuxième surface(s) est(sont) fournie(s) sur un support solide qui est planaire, particulaire ou poreux.

16. Procédé selon l'une quelconque des revendications précédentes, dans lequel l'analyte d'intérêt est choisi dans le groupe composé : d'hormones stéroïdes, d'hormones protéiques, d'acides nucléiques, de peptides, d'antigènes bactériens ou viraux et d'immunoglobulines.


### SEQUENCE LISTING

### (1) GENERAL INFORMATION:

(i) APPLICANT:
   (A) NAME: Unilever PLC
   (B) STREET: Unilever House, Blackfriars
   (C) CITY: London
   (E) COUNTRY: United Kingdom
   (F) POSTAL CODE (ZIP): EC4P 4BQ
   (G) TELEPHONE: (01234) 222068
   (H) TELEFAX: (01234) 222633

   (A) NAME: Unilever NV
   (B) STREET: Weena 455
   (C) CITY: Rotterdam
   (E) COUNTRY: Netherlands
   (F) POSTAL CODE (ZIP): 3013 AL
(ii) TITLE OF INVENTION: Improvements in or Relating to Displacement Assays
(iii) NUMBER OF SEQUENCES: 1
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: Patent In Release #1.0. Version #1.30 (EPO)

### (2) INFORMATION FOR SEQ ID NO: 1:

(i) SEQUENCE CHARACTERISTICS:
   (A) LENGTH: 26 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 1:
   Thr Val Thr Val Ser Ser Gly Gly Gly Gly Ser Gly Gly Gly Gly Ser
   1               5                   10                  15
   Gly Gly Gly Gly Ser Asp Ile Glu Leu Thr
               20                  25